# EUROPEAN PATENT APPLICATION

(11) **EP 1 460 560 A1**
(43) Date of publication of application: **22.09.2004**
(21) Application number: 02792061.0
(22) Date of filing: 27.12.2002
(51) Int. Cl.: G06F 17/30, G06F 19/00

(54) **APPARATUS FOR PREDICTING INTERACTION SITE, METHOD OF PREDICTING INTERACTION SITE, PROGRAM AND RECORDING MEDIUM**

(30) Priority: 27.12.2001 JP 2001398570
(71) Applicant: Celestar Lexico-Sciences, Inc., Chiba-shi, Chiba 261-8501 (JP)
(72) Inventor: SAITO, Seiji c/o Celestar Lexico-Sciences, Inc., Chiba-shi, Chiba 261-8501 (JP)
(74) Representative: HOFFMANN EITLE
(86) International application number: PCT/JP2002/013833
(87) International publication number: WO 2003/056462

(57) **Abstract**

A user inputs target sequence data (10) that is primary sequence information on a target protein to an interaction site prediction apparatus of this invention. A fragment structure prediction simulation is executed to the target sequence data (10) input to each of fragment structure prediction programs (20a to 20d) for predicting a fragment structure of the protein from the primary sequence information on the protein. Fragment structure prediction results (30a to 30d) of the respective fragment structure prediction programs (20a to 20d) are compared with one another (60). Based on this comparison result, a frustration of a local part of the primary sequence information on the target protein is calculated (70). An interaction site in the target protein is predicted based on the calculated frustration of the local part (80).

## Description

### TECHNICAL FIELD

The present invention relates to an interaction site prediction apparatus, an interaction site prediction method, a program, and a recording medium. More specifically, the present invention relates to an interaction site prediction apparatus and an interaction site prediction method, a program, and a recording medium for predicting interaction sites based on frustrations of local sites.

### BACKGROUND ART

It is necessary that a protein has some interaction with the other protein, a substrate, or the like so that the protein acts, that is, the protein exhibits a certain function. To determine an interaction site in the proteins is, therefore, quite a significant theme of study in the fields of drug developments and the like. In the field of bioinformatics and the others, a technique for analyzing interaction sites of a protein by a method of executing a motif search to primary sequence information (amino acid sequence information) on the protein or the like has been conventionally developed. Namely, the interaction sites in the protein are predicted by searching for amino acid sequences that are specifically present in known interaction sites.

The conventional interaction site analysis method based on the motif search or the like has, however, a fundamental disadvantage in terms of system structure. That is, although the known interaction sites can be analyzed, unknown interaction sites cannot be analyzed. The content of this disadvantage will be explained more specifically.

The conventional interaction site analysis method is to register primary sequences specific to interaction sites known in advance in a motif database or the like, and to predict interaction sites using the information. Therefore, with the conventional method, interaction sites that. have not discovered so far cannot be analyzed. For this reason, it is necessary to use an entirely different method from the conventional method when predicting interaction sites that are unknown and not discovered so far on a computer by means of a bioinformatics technique. No effective methods have been, however, established yet.

Meanwhile, a native tertiary structure of a protein is formed so as to eliminate a frustration in an interaction between amino acids as much as possible. Namely, it is said that an energy surface for a protein is designed into a folding funnel so as to provide an overall structure (a native structure) without frustrations. Although the native structure is a structure with less frustration, the frustration is not completely eliminated from the native structure because of complexity of the interaction between elements, flexibility, and an evolutional process of the structure, and the like.

Recent computational experiments reveal that the folding funnel-like energy surface of the protein as an evolutional product is substantially not isotropic but has a direction of large frustration and a direction of little frustration (but is anisotropic) (the energy surface for the protein is in the form of an anisotropic funnel). Structurally speaking, it is shown that local structures include large-frustration structures and little-frustration structures. A local structure site with large frustration is a sacrificed structure site to the stability of the overall structure. This site unavoidably has a distorted structure for the stability of the overall structure. That is, this site is a so-called unstable site in the overall structure.

On the other hand, a protein-protein interaction may be interpreted as a process for further stabilization by allowing two proteins having stable overall structures to act on each other. A structural change when the proteins interact with each other will further be explained. If a protein A and a protein B interact with each other, a local structure of the protein A and that of the protein B undergo structural change and are bound together.

Sites regarded as the local structures that have such change will be considered. A local structure which is stable either locally or entirely is unnecessary to further stabilize. A site which is stable entirely but which is unstable locally is considered to be stabilized by being bound with the other protein or the like, and the overall structure is also further stabilized if the sites are further bound with the other sites. Namely, it can be interpreted that a locally unstable structure area is, relatively highly likely, a protein-protein interaction site. By thus predicting locally unstable sites from the primary sequence, there is a probability that interaction site candidates can be searched.

It is said that the tertiary structure of protein is determined solely based on sequence information. This signifies that there is some correlation between a sequence space and a structure space. If the sequence space and the structure space (native structure space) are compared with each other in magnitude, the sequence space is larger than the structure space. This is because even if a sequence changes a little, a structure does not appear to evolutionally change. In other words, the structure is more evolutionally conservative than the sequence.

Further, the recent structure analysis of evolutionally similar proteins reveals that proteins having a similar sequence have a similar overall structure. Considering that the whole is generated by a combination of parts, it can be assumed that the rule of thumb explained above that may possibly apply to the overall structure of the protein may somewhat apply to a part cut out from the protein.

In fact, proteins for which there is a correlation between a local sequence and a local structure, i.e., similar local sequences have similar local structures are present. In recent studies, an overall structure is tried to be assembled from local sequences using the correlation between the local sequence and the local structure.

In the studies disclosed by, for example, Kim T. Simons et al.: "Assembly of Protein Tertiary Structures from Fragments with Similar Local Sequences using Simulated Annealing and Bayesian Scoring Functions" in *J. Mol. Biol. (1997) 268*, pp. 209-225 (hereinafter, "Literature 1") and Christopher Bystroff et al.: "Predection of Local Structure in Proteins Using a Library of Sequence-Structure Motifs" in *J*. *Mol. Biol. (1998) 281*, pp. 565-577 (hereinafter, "Literature 2"), structures corresponding to local sequences are clustered, whereby a wide structure (folding) space can be narrowed and folding simulation calculation time can be, therefore, reduced.

Literature 1 discloses that because of the limitation of the local structure to a specific offset structure by the local sequence, the structure space is narrowed, the structure is similar to the structure of a protein having a similar sequence, a sequence profile is calculated by multiple alignment, and that a proximity to a query sequence is calculated.

Literature 2 discloses that if there is a correlation between a fragment structure and a sequence, then a limited number of structure candidates can be extracted from a fragment sequence tendency, structures are clustered using two structure indexes, and sequences are calculated using the distance of a frequency profile, and that fragments having similar structures are searched from those having similar sequences and clustered, thereby actually creating sequence-structure fragment clusters.

Local sequence-local structure clusters are used to predict local structures having high correlation with sequences. It is considered to be able to specify locally stable sites (sites having strong correlation) and locally unstable sites in the overall structure (from viewpoints of the correlation between the sequence and the structure) based on the magnitude of the correlation (a certainty factor that represents that a similar sequence has the same structure as that of one sequence). The clusters can be classified variously based on datasets, lengths of local sequences, magnitudes of the clusters, or the like.

It can be said that the site having a strong correlation between the local sequences and the local structure is, highly likely, a site the structure of which is determined in the overall structure only based on the local sequence (i.e., a more stable site in the overall structure). On the other hand, the site having a weak correlation is, highly likely, a site the structure of which is not determined solely by the local sequence (i.e., a site the local structure of which is determined according to the overall structure).

Therefore, large-frustration (unstable) local site candidates may include those each having the weak correlation between the local sequence and the local structure, those each having different high certainty factor results in results using various clusters, those each having a structure different from an initial structure that has a high certainty factor and that is predicted after a folding simulation is executed, and those incompatible with surrounding local structures.

The prediction of a fragment structure of such a large-frustration local site is greatly influenced by the difference in processing manners among those methods explained above. In other words, sites or the like to which greater errors occur by the various methods, i.e., accuracies of which are lower are, highly likely, more-frustration local sites. Accordingly, it can be considered to be able to predict local sites having relatively large frustration through the comparison of results of fragment structure prediction by various methods.

Further, if tertiary structure data on the protein is known, that is, the tertiary structure data on the protein is registered in an existing PDB or the like, the overall structure of the protein is known. Therefore, it is considered to be able to discover local sites (sites each having a high probability of being an interaction site) more clearly by checking the difference between the prediction results of the various fragment structure prediction methods and the actual structure of the protein.

It is, therefore, an object of the present invention to provide an interaction site prediction apparatus, an interaction site prediction method, a program, and a recording medium capable of effectively predicting an interaction site by discovering a local site having frustration from primary sequence information on a protein.

### DISCLOSURE OF THE INVENTION

An interaction site prediction apparatus according to one aspect of the present invention includes: an input unit that inputs primary sequence information on a target protein; a fragment structure prediction program execution unit that allows a fragment structure prediction program for predicting a fragment structure of the target protein from the primary sequence information on the target protein to execute a fragment structure prediction simulation to the primary sequence information input by the input unit; a prediction result comparison unit that compares a fragment structure prediction result of the fragment structure prediction program allowed to execute by the fragment structure prediction program execution unit with the fragment structure prediction result of the other fragment structure prediction program; a frustration calculation unit that calculates a frustration of a local part of the primary sequence information on the target protein based on a comparison result of the prediction result comparison unit; and an interaction site prediction unit that predicts an interaction site in the target protein based on the frustration of the local part calculated by the frustration calculation unit.

According to this apparatus, primary sequence information on a target protein is input, a fragment structure prediction program for predicting a fragment structure of the target protein from the primary sequence information on the target protein is allowed to execute a fragment structure prediction simulation to the input primary sequence information, a fragment structure prediction result of the fragment structure prediction program is compared with the fragment structure prediction result of the other fragment structure prediction program, a frustration of a local part of the primary sequence information on the target protein is calculated based on a comparison result, and an interaction site in the target protein is predicted based on the calculated frustration of the local part. Therefore, it is possible to effectively predict the interaction site by discovering the local site having frustration in the primary site information on the protein.

An interaction site prediction apparatus according to another aspect of the present invention includes: an input unit that inputs primary sequence information on a target protein; a tertiary structure data acquisition unit that acquires tertiary structure data on the target protein; a fragment structure prediction program execution unit that allows a fragment structure prediction program for predicting a fragment structure of the target protein from the primary sequence information on the target protein to execute a fragment structure prediction simulation to the primary sequence information input by the input unit; a prediction result comparison unit that compares a fragment structure prediction result of the fragment structure prediction program allowed to execute by the fragment structure prediction program execution unit with the tertiary structure data acquired by the tertiary structure data acquisition unit; a frustration calculation unit that calculates a frustration of a local part of the primary sequence information on the target protein based on a comparison result of the prediction result comparison unit; and an interaction site prediction unit that predicts an interaction site in the target protein based on the frustration of the local part calculated by the frustration calculation unit.

According to this apparatus, primary sequence information on a target protein is input, tertiary structure data on the target protein is acquired, a fragment structure prediction program for predicting a fragment structure of the target protein from the primary sequence information on the target protein is allowed to execute a fragment structure prediction simulation to the input primary sequence information, a fragment structure prediction result of the fragment structure prediction program is compared with the acquired tertiary structure data, a frustration of a local part of the primary sequence information on the target protein is calculated based on a comparison result, and an interaction site in the target protein is predicted based on the calculated frustration of the local part. Therefore, it is possible to more clearly find the local site (site having a high probability of being an interaction site) by checking the difference between the prediction result of the fragment structure prediction program and the actual fragment structure of the target protein.

The interaction site prediction apparatus according to still another aspect of the present invention, further includes: a certainty factor information setting unit that sets certainty factor information indicating a certainty factor for the fragment structure prediction result of the fragment structure prediction program, wherein the frustration calculation unit calculates the frustration of the local part based on the certainty factor information set by the certainty factor information setting unit and on the comparison result.

This feature illustrates one example of the frustration calculation more specifically. According to this apparatus, certainty factor information indicating a certainty factor for the fragment structure prediction result of the fragment structure prediction program is set, and the frustration of the local part is calculated based on the certainty factor information thus set and on the comparison result. Therefore, it is possible to reflect the certainty factors for the simulation results in the frustration calculation by giving a heavy weight to the fragment structure prediction result data on the program having high certainty factor information (i.e., having a high simulation accuracy),.

An interaction site prediction method according to one aspect of the present invention includes: an input step that inputs primary sequence information on a target protein; a fragment structure prediction program execution step that allows a fragment structure prediction program for predicting a fragment structure of the target protein from the primary sequence information on the target protein to execute a fragment structure prediction simulation to the primary sequence information input by the input step; a prediction result comparison step that compares a fragment structure prediction result of the fragment structure prediction program allowed to execute by the fragment structure prediction program execution step with the fragment structure prediction result of the other fragment structure prediction program; a frustration calculation step that calculates a frustration of a local part of the primary sequence information on the target protein based on a comparison result of the prediction result comparison step; and an interaction site prediction step that predicts an interaction site in the target protein based on the frustration of the local part calculated by the frustration calculation step.

According to this method, primary sequence information on a target protein is input, a fragment structure prediction program for predicting a fragment structure of the target protein from the primary sequence information on the target protein is allowed to execute a fragment structure prediction simulation to the input primary sequence information, a fragment structure prediction result of the fragment structure prediction program is compared with the fragment structure prediction result of the other fragment structure prediction program, a frustration of a local part of the primary sequence information on the target protein is calculated based on a comparison result, and an interaction site in the target protein is predicted based on the calculated frustration of the local part. Therefore, it is possible to effectively predict the interaction site by discovering the local site having frustration in the primary site information on the protein.

An interaction site prediction method according to another aspect of the present invention includes: an input step that inputs primary sequence information on a target protein; a tertiary structure data acquisition step that acquires tertiary structure data on the target protein; a fragment structure prediction program execution step that allows a fragment structure prediction program for predicting a fragment structure of the target protein from the primary sequence information on the target protein to execute a fragment structure prediction simulation to the primary sequence information input by the input step; a prediction result comparison step that compares a fragment structure prediction result of the fragment structure prediction program allowed to execute by the fragment structure prediction program execution step with the tertiary structure data acquired by the tertiary structure data acquisition step; a frustration calculation step that calculates a frustration of a local part of the primary sequence information on the target protein based on a comparison result of the prediction result comparison step; and an interaction site prediction step that predicts an interaction site in the target protein based on the frustration of the local part calculated by the frustration calculation step.

According to this method, primary sequence information on a target protein is input, tertiary structure data on the target protein is acquired, a fragment structure prediction program for predicting a fragment structure of the target protein from the primary sequence information on the target protein is allowed to execute a fragment structure prediction simulation to the input primary sequence information, a fragment structure prediction result of the fragment structure prediction program is compared with the acquired tertiary structure data, a frustration of a local part of the primary sequence information on the target protein is calculated based on a comparison result, and an interaction site in the target protein is predicted based on the calculated frustration of the local part. Therefore, it is possible to more clearly find the local site (site having a high probability of being an interaction site) by checking the difference between the prediction result of the fragment structure prediction program and the actual fragment structure of the target protein.

The interaction site prediction method according to still another aspect of the present invention, further includes: a certainty factor information setting step that sets certainty factor information indicating a certainty factor for the fragment structure prediction result of the fragment structure prediction program, wherein the frustration calculation step calculates the frustration of the local part based on the certainty factor information set by the certainty factor information setting step and on the comparison result.

This feature illustrates one example of the frustration calculation more specifically. According to this method, certainty factor information indicating a certainty factor for the fragment structure prediction result of the fragment structure prediction program is set, and the frustration of the local part is calculated based on the certainty factor information thus set and on the comparison result. Therefore, it is possible to reflect the certainty factors for the simulation results in the frustration calculation by giving a heavy weight to the fragment structure prediction result data on the program having high certainty factor information (i.e., having a high simulation accuracy),.

A computer program that makes a computer to execute an interaction site prediction method according to one aspect of the present invention includes: an input step that inputs primary sequence information on a target protein; a fragment structure prediction program execution step that allows a fragment structure prediction program for predicting a fragment structure of the target protein from the primary sequence information on the target protein to execute a fragment structure prediction simulation to the primary sequence information input by the input step; a prediction result comparison step that compares a fragment structure prediction result of the fragment structure prediction program allowed to execute by the fragment structure prediction program execution step with the fragment structure prediction result of the other fragment structure prediction program; a frustration calculation step that calculates a frustration of a local part of the primary sequence information on the target protein based on a comparison result of the prediction result comparison step; and an interaction site prediction step that predicts an interaction site in the target protein based on the frustration of the local part calculated by the frustration calculation step.

According to this program, primary sequence information on a target protein is input, a fragment structure prediction program for predicting a fragment structure of the target protein from the primary sequence information on the target protein is allowed to execute a fragment structure prediction simulation to the input primary sequence information, a fragment structure prediction result of the fragment structure prediction program is compared with the fragment structure prediction result of the other fragment structure prediction program, a frustration of a local part of the primary sequence information on the target protein is calculated based on a comparison result, and an interaction site in the target protein is predicted based on the calculated frustration of the local part. Therefore, it is possible to effectively predict the interaction site by discovering the local site having frustration in the primary site information on the protein.

A computer program that makes a computer to execute an interaction site prediction method according to another aspect of the present invention includes: an input step that inputs primary sequence information on a target protein; a tertiary structure data acquisition step that acquires tertiary structure data on the target protein; a fragment structure prediction program execution step that allows a fragment structure prediction program for predicting a fragment structure of the target protein from the primary sequence information on the target protein to execute a fragment structure prediction simulation to the primary sequence information input by the input step; a prediction result comparison step that compares a fragment structure prediction result of the fragment structure prediction program allowed to execute by the fragment structure prediction program execution step with the tertiary structure data acquired by the tertiary structure data acquisition step; a frustration calculation step that calculates a frustration of a local part of the primary sequence information on the target protein based on a comparison result of the prediction result comparison step; and an interaction site prediction step that predicts an interaction site in the target protein based on the frustration of the local part calculated by the frustration calculation step.

According to this program, primary sequence information on a target protein is input, tertiary structure data on the target protein is acquired, a fragment structure prediction program for predicting a fragment structure of the target protein from the primary sequence information on the target protein is allowed to execute a fragment structure prediction simulation to the input primary sequence information, a fragment structure prediction result of the fragment structure prediction program is compared with the acquired tertiary structure data, a frustration of a local part of the primary sequence information on the target protein is calculated based on a comparison result, and an interaction site in the target protein is predicted based on the calculated frustration of the local part. Therefore, it is possible to more clearly find the local site (site having a high probability of being an interaction site) by checking the difference between the prediction result of the fragment structure prediction program and the actual fragment structure of the target protein.

The program according to still another aspect of the present invention, further includes: a certainty factor information setting step that sets certainty factor information indicating a certainty factor for the fragment structure prediction result of the fragment structure prediction program, wherein the frustration calculation step calculates the frustration of the local part based on the certainty factor information set by the certainty factor information setting step and on the comparison result.

This feature illustrates one example of the frustration calculation more specifically. According to this program, certainty factor information indicating a certainty factor for the fragment structure prediction result of the fragment structure prediction program is set, and the frustration of the local part is calculated based on the certainty factor information thus set and on the comparison result. Therefore, it is possible to reflect the certainty factors for the simulation results in the frustration calculation by giving a heavy weight to the fragment structure prediction result data on the program having high certainty factor information (i.e., having a high simulation accuracy),.

Furthermore, the present invention relates to the recording medium. The recording medium according to the present invention records the program explained above.

This recording medium can realize the program using a computer by allowing the computer to read each program recorded on the recording medium, and can exhibit the same advantages as those of the program.

### BRIEF DESCRIPTION OF THE DRAWINGS

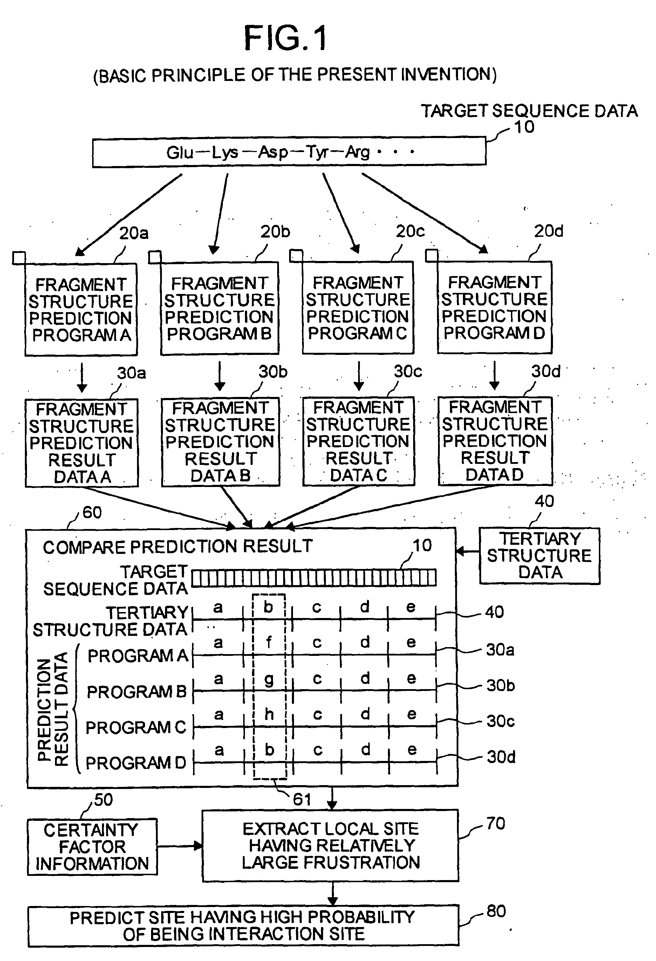

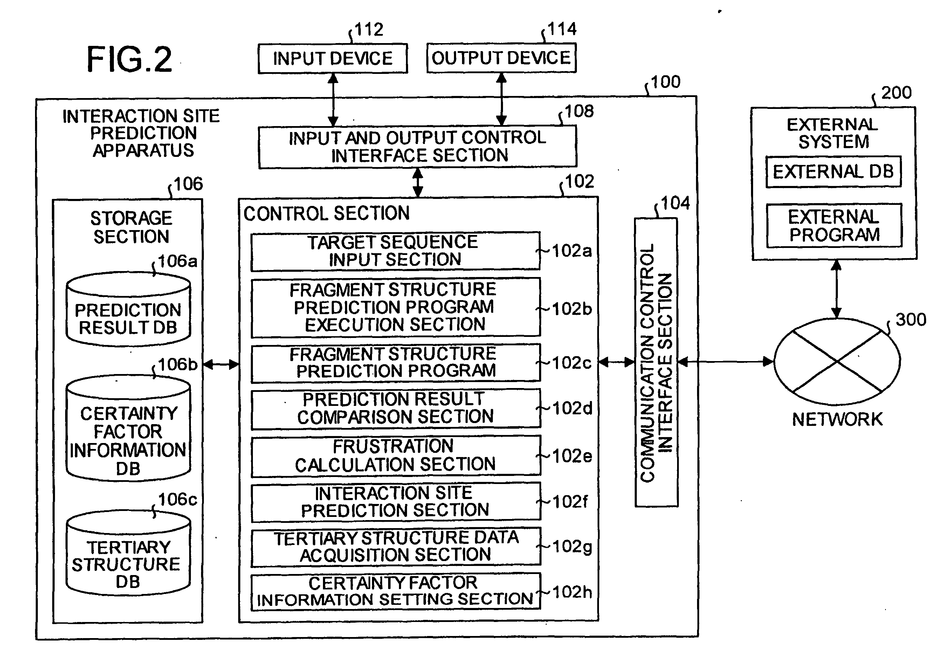

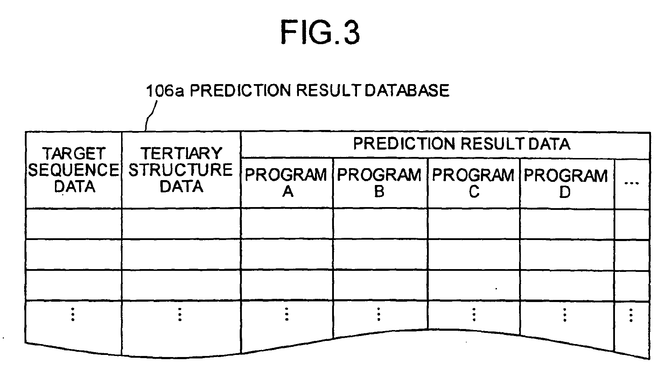

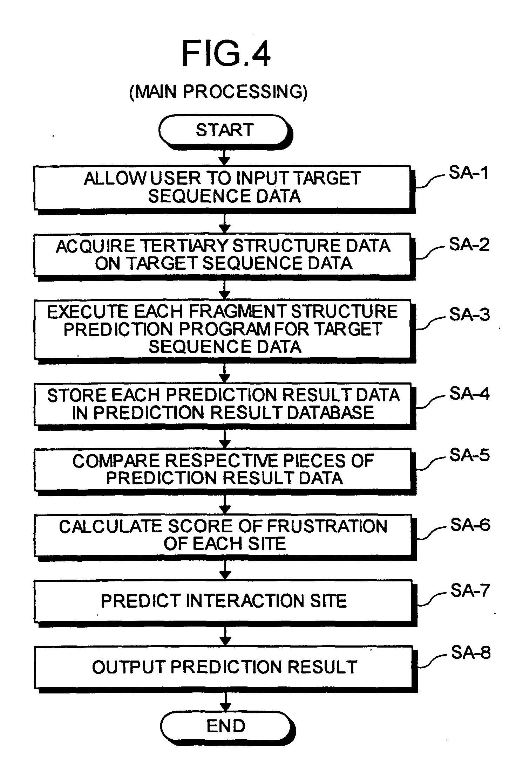

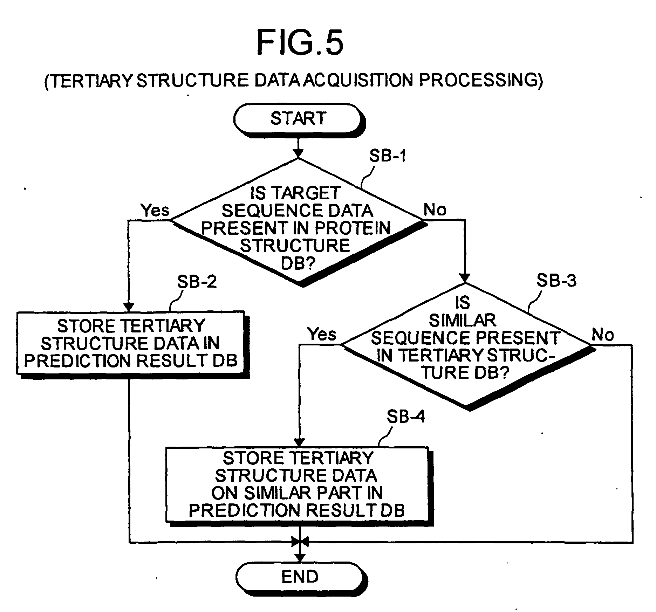

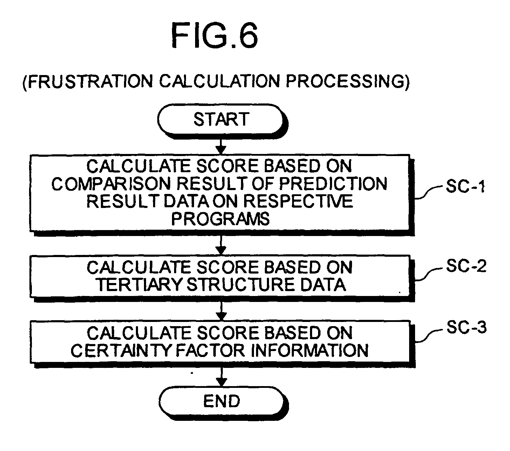

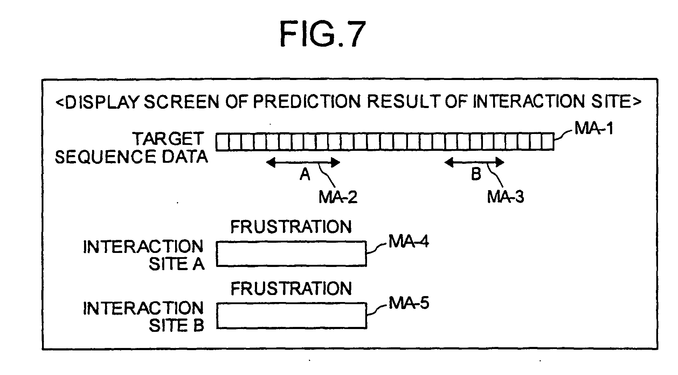

Fig. 1 is a principle block diagram which illustrates the basic principle of the present invention; Fig. 2 is a block diagram which illustrates one example of the configuration of a system to which the present invention is applied; Fig. 3 is a flow chart which illustrates one example of information stored in a prediction result database 106a; Fig. 4 is a flow chart which illustrates one example of a main processing performed by the system according to one embodiment of the present invention; Fig. 5 is a flow chart which illustrates one example of a protein data acquisition processing performed by the system according to the embodiment; Fig. 6 is a flow chart which illustrates one example of a frustration execution processing executed by a frustration calculation section 102e in the system; and Fig. 7 illustrates one example of a display screen of an interaction site prediction result displayed on an output device 114 for an interaction site prediction apparatus 100.

### BEST MODE FOR CARRYING OUT THE INVENTION

Exemplary embodiments of an interaction site prediction apparatus, an interaction site prediction method, a program, and a recording medium according to the present invention will be explained hereinafter in detail based on the drawings. It should be noted that the present invention is not limited to the embodiment.

### [Outline of the Present Invention]

The outline of the present invention will be explained first, followed by the configuration, processings, and the like of the present invention. Fig. 1 is a principle block diagram which illustrates the basic principle of the present invention.

Schematically, the present invention has the following basic features. The present invention characteristically predicts an interaction site using a fragment structure cluster, predicts an interaction site from a fragment structure prediction result, predicts an interaction site from a difference in prediction result among various fragment structure predictions, predicts that a site that has a difference in fragment structure prediction result is a stress site having large frustration in an overall structure and a local structure, predicts that a structural stress site (relatively structurally unstable site) is likely to be a site interacting with the other site, etc. The basic principle of the present invention will now be explained.

First of all, a user inputs target sequence data 10 that is primary sequence information on a target protein to the interaction site prediction apparatus according to the present invention. This target sequence data 10 may be input by, for example, user's selecting one primary sequence information registered in an external database such as SWISS-PROT, PIR, or TrEMBL, or by directly inputting desired primary sequence information.

The interaction site prediction apparatus according to the present invention then executes a fragment structure prediction simulation to the target sequence data 10 input to respective fragment structure prediction programs 20a to 20d for predicting a fragment structure of the target protein from the primary sequence information on the protein. Each of the fragment structure prediction programs 20a to 20d executes the fragment structure prediction simulation using, for example, the method disclosed by Literature 1 or 2, a threading method, or an Ab initio method.

The interaction site prediction apparatus according to the present invention compares fragment structure prediction results 30a to 30d of the respective fragment structure prediction programs 20a to 20d (60). Namely, the interaction site prediction apparatus puts the execution results of the respective prediction programs corresponding to the target sequence data 10 in parallel, and compares the results (30a to 30d) with one another.

The interaction site prediction apparatus according to the present invention calculates frustrations of local parts of the primary sequence information on the target protein based on this comparison result (70). Namely, the interaction site prediction apparatus extracts local parts for which different fragment structures are predicted based on the respective prediction result data (30a to 30b) from the comparison result, and calculates frustrations of the parts. The existing fragment structure prediction programs 20a to 20d make predictions basically from part of local sequences in the primary sequence information. However, since a fragment structure is finally determined based on the relationship with the overall structure of the protein, fragment structure prediction results are not often right in the sites in which there is no compatibility between the whole and the local part, i.e., local sites having large frustration. It is, therefore, possible to assume that the local parts having different prediction results among a plurality of programs have large frustration.

A frustration calculation method may be executed as follows. A frustration may be increased or decreased according to the number of fragment structure prediction programs that output different pieces of prediction result data, the frustration may be increased or decreased according to an average, a distribution value or the like of certainty factors for the respective structures of the different prediction results, or the frustration may be calculated by calculating an energy quantity of an amino acid sequence in the site using a molecular mechanics-bases method, a molecular dynamics-basis method, or the like.

The interaction site prediction apparatus according to the present invention predicts interaction sites in the target protein based on the calculated frustrations of the local parts (80). Namely, the apparatus predicts, for example, a local part (61), in which a frustration exceeding a certain threshold is present, as an interaction site.

In addition, if the tertiary structure data on the target protein is registered in the external database such as PDB or SCOP, the interaction site prediction apparatus according to the present invention acquires the tertiary structure data 40 and uses the data 40 in the comparison of prediction results. Namely, the interaction site prediction apparatus compares the tertiary structure data 40 on the actual tertiary structure of the target protein with the prediction result data 30a to 30d on the respective prediction programs.

The apparatus calculates a high frustration for a site in which the actual tertiary structure data 40 differs from the prediction program prediction result data 30a to 30d. If the tertiary structure on the protein is known, i.e., the tertiary structure of the protein is registered in the existing PDB or the like, the overall structure of the protein is known. Therefore, based on the difference between the prediction results of various fragment structure prediction methods and the actual structure of the protein, it is possible to find local sites having frustration (local site each having a high probability of being an interaction site) more clearly. For example, the frustration may be increased or decreased according to the number of fragment structure prediction programs that output the prediction result data different from the actual tertiary structure data 40.

Further, the interaction site prediction apparatus according to the present invention sets certainty factor information 50 that indicates certainty factors of the fragment structure prediction data 30a to 30d on the respective fragment structure prediction programs 20a to 20d. Namely, the apparatus sets simulation accuracies of the respective fragment structure prediction programs 20a to 20d based on the actual tertiary structure or the like.

The interaction site prediction apparatus according to the present invention calculates the frustrations of the local regions based on the certainty factor information 50 thus set and the comparison result. Namely, by giving a heavy weight to the fragment structure prediction result data on the program having high certainty factor information (i.e., having a high simulation accuracy), the certainty factors for the simulation results can be reflected in the frustration calculation.

That is, according to the present invention, the structure prediction results of the respective methods and the certainty factors for the structures are analyzed. Scores relating to site candidates are calculated so that a site having a low correlation between the local sequence and the local structure, a site for which results using various clusters show different high certainty factors, a site which has a structure different from the predicted initial structure with a high certainty factor after a folding simulation is executed, a site incompatible with surrounding local structures, and the like have large frustration. The sites each having a high probability of being an interaction site are given scores in a descending order of probability based on the calculated results. The sites can be thereby extracted.

### [System Configuration]

The configuration of a system to which the present invention is applied will be explained. Fig. 2 is a block diagram which illustrates one example of the configuration of the system to which the present invention is applied. Fig. 2 conceptually illustrates only sections relating to the present invention in the configuration. The system is schematically constituted so that an interaction site prediction apparatus 100 and an external system 200 that provides an external database on sequence information, a tertiary structure, and the like, and external programs for homology search, the fragment structure prediction, and the like are communicably connected to each other through a network 300.

In Fig. 2, the network 300 functions to connect the interact site prediction apparatus 100 and the external system 200 to each other, and is, for example, the Internet.

In Fig. 2, the external system 200 is connected to the interact site prediction apparatus 100 through the network 300, and functions to provide external databases about sequence information, tertiary structures, and the like, and a website for executing external programs for a homology search, a motif search, fragment structure prediction, and the like.

The external system 200 may be constituted as a WEB server, an ASP server, or the like, and hardware of the external system 200 may include an information processing apparatus such as a commercially available workstation or personal computer, and accessories of the apparatus. Respective functions of the external system 200 are realized by a CPU, a disk device, a memory device, an input device, an output device, a communication control device, and the like in the hardware configuration of the external system 200 as well as programs for controlling these devices, and the like.

In Fig. 2, the interaction site prediction apparatus 100 schematically includes a control section 102 such as the CPU for generally controlling entirety of the interaction site prediction apparatus 100, a communication control interface section 104 connected to a communication device (not illustrated) such as a router connected to a communication line or the like, an input and output control interface 108 connected to an input device 112 and an output device 114, and a storage section 106 that stores various databases and tables (a prediction result database 106a to a protein structure database 106c). The respective sections are communicably connected to one another through arbitrary communication lines. In addition, this interaction site prediction apparatus 100 is communicably connected to the network 300 through the communication device such as the router and a wired or wireless communication line such as a dedicated line.

In Fig. 2, the various databases and tables (the prediction result database 106a to the protein structure database 106c) are storage units such as fixed disk devices or the like, and store various programs, tables, files, databases, webpage files, and the like used for various processings.

Among the constituent elements of the storage section 106, the prediction result database 106a is a prediction result storage unit that stores information on prediction results of the respective fragment structure prediction programs. Fig. 3 illustrates one example of the information stored in the prediction result database 106a.

As illustrated in Fig. 3, the information stored in the prediction result database 106a is constituted so as to make target sequence data that is primary sequence information (amino acid sequence information) on the target protein, tertiary structure data on the target sequence data acquired from the protein structure database, and prediction result data on the respective fragment structure prediction programs correspond to one another.

A certainty factor information database 106b is a prediction result information storage unit that stores certainty factor information that indicates a certainty factor for fragment structure prediction result data on each fragment structure prediction program. For example, a standard value of an accuracy of a simulation result (when the simulation accuracy that is a rate of coincidence between one fragment structure prediction result and actual tertiary structure data is, for example, 60 percents) is one. If the accuracy is higher than the standard value, the certainty factor may be set higher according to the accuracy. If the accuracy is lower than the standard value, the certainty factor may be set lower according to the accuracy. In addition, the certainty factor may be set for every fragment structure prediction program, every structure, or every amino acid in each sequence. That is, when a certain fragment structure prediction program predicts a fragment structure of a certain amino acid in a certain sequence, a certainty factor that the structure is a structure "a", a certainty factor that the structure is a structure "b", and the like may be individually set.

The protein structure database 106c is a database that stores tertiary structure data on proteins. The protein structure database 106c may be an external protein structure database accessed through the Internet, or an in-house database created by copying the database, by storing original sequence information, or by adding individual annotation information and the like to the database.

In Fig. 2, the communication control interface section 104 controls the communication between the interaction site prediction apparatus 100 and the network 300 (or the communication device such as the router). Namely, the communication control interface 104 functions to communicate data with other terminals through communication lines.

In Fig. 2, the input and output control interface section 108 controls the input device 112 and the output device 114. As the output device 114, a monitor (including a home television set), a loudspeaker or the like can be used (it is noted that the output device 114 is sometimes referred to as "monitor" hereafter). As the input device 112, a keyboard, a mouse, a microphone, or the like can be used. The monitor also realizes a pointing device function in cooperation with the mouse.

In Fig. 2, the control section 102 includes an internal memory for storing various programs such as an OS (Operating System), programs for specifying various processing procedures, and required data. Using these programs and the like, information processings for executing various processings are performed. The control section 102 functionally conceptually includes a target sequence input section 102a, a fragment structure prediction program execution section 102b, a fragment structure prediction program 102c, a prediction result comparison section 102d, a frustration calculation section 102e, an interaction site prediction section 102f, a tertiary structure data acquisition section 102g, and a certainty factor information setting section 102h.

Among the constituent elements of the control section 102, the target sequence input section 102a is an input unit that inputs the primary sequence information (target sequence data) on the target protein. The fragment structure prediction program execution section 102b is a fragment structure prediction program execution unit that allows each fragment structure prediction program to execute a fragment structure prediction simulation to the primary sequence information (target sequence data) input by the input unit. The fragment structure prediction program 102c is a fragment structure prediction program for predicting the fragment structure of the protein from the primary information on the protein.

The prediction result comparison section 102d is a prediction result comparison unit that compares fragment structure prediction results of the respective fragment structure prediction programs with one another, and is a prediction result comparison unit that compares the fragment structure prediction results of the respective fragment structure prediction programs with the tertiary structure data acquired by the tertiary structure data acquisition unit. The frustration calculation section 102e is a frustration calculation unit that calculates frustrations of local parts of the primary sequence information (target sequence data) on the target protein, and is a frustration calculation unit that calculates the frustration of the local part based on the certainty factor information set by the certainty factor information setting unit and the comparison results.

The interaction site prediction section 102f is an interaction site prediction unit that predicts interaction sites in the target protein based on the frustrations of the local parts calculated by the frustration calculation unit. The tertiary structure data acquisition section 102g is a tertiary structure data acquisition unit that acquires the tertiary structure data on the target protein. The certainty factor information setting section 102h is a certainty factor information setting unit that sets the certainty factor information that indicates a certainty factor for the fragment structure prediction result of each fragment structure prediction program. Details of processings performed by the respective sections will be explained later.

### [System Processings]

One example of processings performed by the system according to the embodiment constituted as explained above will next be explained with reference to Figs. 4 to 7.

### [Main Processing]

The details of a main processing will be explained with reference to Fig. 4. Fig. 4 is a flow chart which illustrates one example of the main processing performed by the system according to the embodiment.

The interaction site prediction apparatus 100 allows the user to input the primary sequence information (target sequence data) on the target protein by a processing performed by the target sequence input section 102a (at a step SA-1).

The interaction site prediction apparatus 100 acquires the tertiary structure data on the target sequence data input by the user by a processing performed by the tertiary structure data acquisition section 102g (at a step SA-2).

The detail of the tertiary structure data acquisition processing performed by the tertiary structure data acquisition section 102g at the step SA-2 will be explained with reference to Fig. 5. Fig. 5 illustrates one example of the tertiary structure data acquisition processing performed by the system according to the embodiment.

The tertiary structure data acquisition section 102g determines whether target sequence data is registered while referring to the protein structure database 106c (at a step SB-1). If it is determined at the step SB-1 that the target sequence data is registered in the protein structure database 106c, the tertiary structure data acquisition section 102g acquires the tertiary structure data on the target sequence data from the protein structure database 106c, and stores the acquired tertiary structure data in a predetermined storage area of the prediction result database 106a (at a step SB-2).

If it is determined at the step SB-1 that the target sequence data is not registered in the protein structure database 106c, the tertiary structure data acquisition section 102g determines whether tertiary structure data on a protein having a similar sequence to the target sequence data is present in the protein structure database 106c (at a step SB-3). Namely, the tertiary structure data acquisition section 102g compares the target sequence data with sequence data corresponding to each protein having a known structure and registered in the protein structure database 106c using a program for determining a homology between sequences, and determines whether sequence data having a high homology (which data may correspond to part of the target sequence data) is present.

If it is determined at the step SB-3 that the tertiary structure data on the protein having a similar sequence to the target sequence data is present in the protein structure database 106c, the tertiary structure data acquisition section 102g stores the tertiary structure data on the similar part in the predetermined storage area of the prediction result database 106a (at a step SB-4). If the tertiary structure data is present for part of the target sequence data, the tertiary structure data on a part in which the tertiary structure data is present is stored in the prediction result database 106a.

If it is determined at the step SB-3 that the tertiary structure data on the protein having a similar sequence to the target sequence data is not present in the protein structure database 106c, the tertiary structure data acquisition processing is finished.

Referring back to Fig. 4, the interaction site prediction apparatus 100 allows one or two or more fragment structure prediction programs 102c to execute the target sequence data by a processing performed by the fragment structure prediction program execution section 102b (at a step SA-3). Namely, the fragment structure prediction program execution section 102b makes input forms of the respective fragment structure prediction programs 102c uniform by, for example, converting a format of the target sequence data into a predetermined format or by adding predetermined header information to the target sequence data, and then executes the fragment structure prediction programs 102c. The fragment structure prediction programs 102c may be programs present in the interaction site prediction apparatus 100 or the external programs in the external system 200 which programs can be executed remotely by the section 102b through the network 300.

The fragment structure prediction program execution section 102b may obtain the fragment structure by one of the following methods.

### (1) Ortholog homology analysis

An ortholog homology analysis is an analysis method for interpreting that a probability that certain genes X and Y interact with each other is high if orthologs X' and Y' of the certain genes X and Y are present and it is known that X' and Y' interact with each other.

### (2) Rozetta Stone Method

A Rozetta stone method is an analysis method for interpreting that, if a first half of a gene Z, which is a gene of a biological species different from a certain biological species having genes X and Y, is similar to X and a second half of the gene Z is similar to Y, the gene Z which was previously one gene is separated to X and Y by evolution, and that the probability that genes X and Y of a certain biological species interact with each other is high.

### (3) Threading Method

A threading method is for making structure prediction by creating a 3D profile from structure information and aligning the sequence to the structure using the 3D profile.

### (4) Topology Fingerprint Method

A topology fingerprint method is for performing alignment between a certain protein A and a protein B by extracting a parameter for the structure of the certain protein A referred to as "topology print" (corresponding to a coefficient of an energy function for the structure or the like) using internal and external properties of the protein A, a distance map of the structure or the like, and by applying this parameter to a sequence in the protein B.

### (5) Motif Search

A motif search is a method for predicting a function (structure) of a protein by searching for the protein using a motif thereof registered in a motif registration database (e.g., PROSITE or Pfam) as a key. It may be considered that a probability that proteins having the same functional motif have the same function (structure) is high.

### (6) Module Search (3D keynote) method

A module is an amino acid sequence having a compact structure in a spherical protein and consisting of about 15 residues. It is said that an intron has a good correspondence to a boundary of modules. A module search method is, based on this knowledge, for making structure prediction by extracting an amino acid sequence pattern from a common module structure present in various proteins, and by searching for the structure. It is considered that a similarity among characteristic amino acid sequences extracted from module structures related to functions signifies a functional similarity among these proteins.

### (7) Evolutionary Trace Method

An evolutionary trace method is for predicting a substrate-binding site or an interaction site with another protein by evolutionally tracing a change in sequence if multiple alignment with respect to the tertiary structure of a certain protein and that of an ortholog of the certain protein is performed.

### (8) Homology Profile Method (hereinafter, "HMM method")

If a certain sequence A is present, relevant proteins of a protein having the sequence A such as a family of the protein and an ortholog thereof are present, and alignment with respect to the certain sequence A and the relevant protein sequences is performed, a profile matrix is created with respect to the sequence A or the family of the sequence A. An HMM method is for performing alignment between the profile matrix and a sequence B (or a profile of the sequence B obtained by similarly creating a profile matrix of the sequence B). With this method, it is possible to search for farther relations than the alignment between the sequences A and B.

### (9) Fugue

Fugue is a method for classifying amino acid stock properties of analogous proteins depending on in which part an amino acid of each protein is stocked in the tertiary structure, and for creating a structure-sequence substitution matrix, such as BLOSUM, higher in sensitivity than a conventional substitution matrix using the classification information. With this method, it is possible to realize alignment with higher sensitivity by performing the alignment using the matrix.

It is noted that the present invention is not limited to these methods and that any fragment structure prediction method may be used for the present invention.

The fragment structure prediction program execution section 102b stores fragment structure prediction results which are simulation results of the respective fragment structure prediction programs 102c in a predetermined storage area of the prediction result database 106a (at a step SA-4).

The interaction site prediction apparatus 100 compares the fragment structure prediction results of the respective fragment structure prediction programs 102c for the target sequence data which are stored in the prediction result database 106a with one another by a processing performed by the prediction result comparison section 102d (at a step SA-5). Namely, the prediction result comparison section 102d compares respective prediction results from a top to an end of the target sequence data for the fragment structure prediction results of the respective fragment structure prediction programs 102c. If the fragment structure prediction program execution section 102b can acquire the tertiary structure data corresponding to the target sequence data at the step SA-2, that is, if the tertiary structure data on the target sequence data is stored in the prediction result database 1 06a, the prediction result comparison section 102d compares the tertiary structure data with the fragment structure prediction results of the respective fragment structure prediction programs 102c.

The interaction site prediction apparatus 100 calculates a score of a frustration of each local part of the target sequence data by a processing performed by the frustration calculation section 102e (at a step SA-6). Fig. 6 is a flow chart which illustrates one example of a frustration execution processing executed by the frustration calculation section 102e in this system.

As illustrated in Fig. 6, the frustration calculation section 102e may calculate the score of the frustration by, for example, increasing or decreasing the score according to the number of the fragment structure prediction programs having different results for the local part for which the fragment structure prediction programs output different fragment structure prediction results, by increasing the frustration according to an average, a distribution value or the like of certainty factors for the structures of the different prediction results, or by calculating an energy quantity of an amino acid sequence by the molecular mechanics-basis method, the molecular dynamics-basis method, or the like and calculating the frustration using the energy quantity for the local part for which the fragment structure prediction programs output different fragment structure results (at a step SC-1).

Further, the frustration calculation section 102e may calculate a score of a high frustration for the part for which the tertiary structure data differs from the fragment structure prediction results of the respective prediction programs (at a step SC-2). For example, the score of each frustration may be increased or decreased according to the number of the fragment structure prediction programs which outputs fragment structure prediction results different from the tertiary structure data.

The frustration calculation section 102e may acquire certainty factor information on the respective fragment structure prediction programs stored in advance by a processing performed by the certainty factor information setting section 102h while referring to the certainty factor information database 106b, and calculate the score of each frustration based on the certainty factor information (at a step SC-3). Namely, the frustration calculation section 102e calculates the score of the frustration while giving a high weight to the fragment structure prediction data on the fragment structure prediction program 102c having a high simulation accuracy.

One example of the setting of the certainty factor information made by the certainty factor information setting section 102h will be explained. The certainty factor information setting section 102h compares the fragment structure prediction result of each fragment structure prediction program 102c with the tertiary structure, and calculates an accuracy (a coincidence rate) of the fragment structure prediction result of each fragment structure prediction program 102c. The certainty factor information setting section 102h sets an average of the accuracies of the respective fragment structure prediction programs 102c as a standard certainty factor information (e.g., one), calculates an accuracy equal to or higher than the average so as to be higher than the standard certainty factor information (e.g., a number greater than one), calculates an accuracy equal to or lower than the average so as to be lower than the standard certainty factor information (e.g., a number smaller than one), and stores the calculated accuracies in a predetermined storage area of the certainty factor information database 106b.

The certainty factor information setting section 102h may set the certainty factor information on each fragment structure prediction program 102c for every amino acid (residue) in each sequence. Namely, the certainty factor information setting section 102h may set the certainty factor information on each fragment structure prediction program 102c for every amino acid in each sequence for a sequence prediction result obtained by each fragment structure prediction program 102c (e.g., as for the first amino acid in a sequence, a program A has certainty factor information on a structure "a" of 1.5, that of a structure "b" of 0.7, and that of a structure c of 1.1).

The certainty factor information setting section may set the certainty factor information on each fragment structure prediction program 102c for every structure. Namely, some fragment structure prediction programs 102c have a high or low accuracy for a specific structure. Therefore, the certainty factor information on each fragment structure prediction program 102c may be set for every structure (e.g., the program A has certainty factor information on the structure "a" of 1.5, that of the structure "b" of 0.7, and that of the structure c of 1.1).

Referring back to Fig. 4, the interaction site prediction apparatus 100 predicts local parts that are likely to be interaction sites in the target sequence data based on the calculated scores of the frustrations of the local parts by a processing performed by the interaction site prediction section 102f (at a step SA-7). Namely, the interaction site prediction section 102f predicts the local parts each having, for example, the frustration score exceeding a certain threshold as interaction sites..

The interaction site prediction apparatus 100 then outputs a prediction result of the interaction sites in the sequence data to the output device 114 (at a step SA-8).

Fig. 7 illustrates one example of a display screen of the interaction site prediction result displayed on the output device 114 of the interaction site prediction apparatus 100. As illustrated in Fig. 7, the display screen of the interaction site prediction result includes a display area MA-1 for sequence information on the target sequence data, display areas MA-2 and MA-3 each for the local part predicted as the interaction site, display areas MA-4 and MA-5 each for the frustration score of each local part predicted as the interaction site, and the like. The main processing is thus finished.

### [Other Embodiments]

The embodiment of the present invention has been explained so far. However, the present invention is not limited to the embodiment but may be carried out by various other embodiments within the scope of the technical spirit according to the appended claims.

For example, the instance in which the interaction site prediction apparatus 100 makes interaction site prediction in a stand-alone fashion has been explained. Alternatively, the interaction site prediction apparatus 100 may make the interaction site prediction in response to a request from a client terminal constituted separately from the interaction site prediction apparatus 100, and may return the processing result to the client terminal.

The respective fragment structure prediction programs may make prediction by any methods.

Further, among the respective processings explained in the embodiment, all of or part of the processings explained to be performed automatically may be performed manually or all of or part of the processings explained to be performed manually may be performed automatically by a well-known method.

The processing procedures, control procedures, specific names, information including various pieces of registered data and parameters for search conditions and the like, screen examples, and database configurations explained above or illustrated in the drawings may be arbitrarily changed unless specified otherwise.

The respective constituent elements of the interaction site prediction apparatus 100 illustrated in the drawings are functionally conceptual, and the interaction site prediction apparatus 100 is not always required to be physically constituted as illustrated in the drawings.

For instance, all of or arbitrary part of the processing functions of the respective servers provided in the interaction site prediction apparatus 100, particularly the respective processing functions performed by the control section can be realized by the CPU (Central Processing Unit) and programs interpreted and executed by the CPU, or can be realized as hardware based on wired logic. The programs are recorded on the recording medium to be explained later, and mechanically read by the interaction site prediction apparatus 100 as needed.

Also, the programs may be recorded in an application program server, which is connected to the interaction site prediction apparatus 100 via an arbitrary network. The programs can be entirely or partially downloaded as needed.

The various databases and the like (the prediction result database 106a to the protein structure database 106c) stored in the storage section 106a are storage units such as memory devices, e.g., a RAM and a ROM, fixed disk devices, e.g., a hard disk, a flexible disk, and an optical disk. They store various programs, tables, files, databases, webpage files, and the like used for various processings and provision of websites.

In addition, the interaction site prediction apparatus 100 may be realized by connecting peripherals such as a printer, a monitor, and an image scanner to an information processing apparatus such as an information processing terminal, e.g., a well-known personal computer or workstation, and by installing software (including a program, data, or the like) for realizing the method of the present invention into the information processing apparatus.

The specific form of distribution and integration of the interaction site prediction apparatus 100 is not limited to that illustrated in the drawings. All of or part of the interaction site prediction apparatus 100 can be functionally or physically distributed or integrated in arbitrary units according to various loads and the like. For example, each database may be constituted independently as an independent database device, and part of the processings may be realized using a CGI (Common Gateway Interface).

Further, the program according to the present invention can be stored in a computer readable recording medium. It is assumed herein that examples of this "recording medium" include arbitrary "portable physical mediums" such as a flexible disk, a magneto-optical disk, a ROM, an EPROM, an EEPROM, a CD-ROM, an MO, and a DVD, arbitrary "fixed physical mediums" such as a ROM, a RAM, and an HD included in various computer systems, and "communication mediums" that temporarily hold the program such as a communication line or a carrier wave used when the program is transmitted through the network represented by a LAN, a WAN, or the Internet.

The "program" is a data processing method described in an arbitrary language or by an arbitrary description method, and the form of the "program" is not limited but may be a source code, a binary code, or the like. The "program" is not limited to a program constituted as a single program. Examples of the "program" include a program constituted to be distributed as a plurality of modules or libraries, and a program that fulfils its function in cooperation with another program represented by the OS (Operating System). The specific configurations, reading procedures, install procedures after reading, and the like of the respective devices shown in the embodiment for reading the recording medium may be well-known configurations and procedures.

Furthermore, the network 300 functions to connect the interaction site prediction apparatus 100 and the external system 200 to each other, and may include any one of, for example, the Internet, the Intranet, a LAN (which may be either wired or wireless), a VAN, a personal computer communication network, a public telephone network (which may be either analog or digital), a dedicated line network (which may be either analog or digital), a CATV network, a portable line exchange network/portable packet exchange network such as an IMT 2000 network, a GSM network, or a PDC/PDC-P network, a wireless call network, a local wireless network such as Bluetooth, and satellite communications network such as CD, BS, or ISDB. That is, the present system can transmit and receive various pieces of data through an arbitrary network whether the system is wired or wireless.

As explained so far in detail, according to the present invention, primary sequence information on a target protein is input, a fragment structure prediction program for predicting a fragment structure of the target protein from the primary sequence information on the target protein is allowed to execute a fragment structure prediction simulation to the input primary sequence information, a fragment structure prediction result of the fragment structure prediction program is compared with the fragment structure prediction result of the other fragment structure prediction program, a frustration of a local part of the primary sequence information on the target protein is calculated based on a comparison result, and an interaction site in the target protein is predicted based on the calculated frustration of the local part. Therefore, it is possible to provide the interaction site prediction apparatus, the interaction site prediction method, the program, and the recording medium capable of effectively predicting the interaction site by discovering the local site having frustration in the primary site information on the protein.

Further, according to the present invention, primary sequence information on a target protein is input, tertiary structure data on the target protein is acquired, a fragment structure prediction program for predicting a fragment structure of the target protein from the primary sequence information on the target protein is allowed to execute a fragment structure prediction simulation to the input primary sequence information, a fragment structure prediction result of the fragment structure prediction program is compared with the acquired tertiary structure data, a frustration of a local part of the primary sequence information on the target protein is calculated based on a comparison result, and an interaction site in the target protein is predicted based on the calculated frustration of the local part. Therefore, it is possible to provide the interaction site prediction apparatus, the interaction site prediction method, the program, and the recording medium capable of more clearly finding the local site (site having a high probability of being an interaction site) by checking the difference between the prediction result of the fragment structure prediction program and the actual fragment structure of the target protein.

In addition, according to the present invention, certainty factor information indicating a certainty factor for the fragment structure prediction result of the fragment structure prediction program is set, and the frustration of the local part is calculated based on the certainty factor information thus set and on the comparison result. Therefore, it is possible to provide the interaction site prediction apparatus, the interaction site prediction method, the program, and the recording medium capable of reflecting the certainty factors for the simulation results in the frustration calculation by giving a heavy weight to the fragment structure prediction result data on the program having high certainty factor information (i.e., having a high simulation accuracy),.

### INDUSTRIAL APPLICABILITY

As explained so far, the interaction site prediction apparatus, the interaction site prediction method, the program, and the recording medium according to the present invention can be used for the prediction of the tertiary structure of a protein and the analysis of the interaction site of the protein as well as drug design and the like using analysis results.

## Claims

1. An interaction site prediction apparatus comprising:
an input unit that inputs primary sequence information on a target protein;
a fragment structure prediction program execution unit that allows a fragment structure prediction program for predicting a fragment structure of the target protein from the primary sequence information on the target protein to execute a fragment structure prediction simulation to the primary sequence information input by the input unit;
a prediction result comparison unit that compares a fragment structure prediction result of the fragment structure prediction program allowed to execute by the fragment structure prediction program execution unit with the fragment structure prediction result of the other fragment structure prediction program;
a frustration calculation unit that calculates a frustration of a local part of the primary sequence information on the target protein based on a comparison result of the prediction result comparison unit; and
an interaction site prediction unit that predicts an interaction site in the target protein based on the frustration of the local part calculated by the frustration calculation unit.

2. An interaction site prediction apparatus comprising:
an input unit that inputs primary sequence information on a target protein;
a tertiary structure data acquisition unit that acquires tertiary structure data on the target protein;
a fragment structure prediction program execution unit that allows a fragment structure prediction program for predicting a fragment structure of the target protein from the primary sequence information on the target protein to execute a fragment structure prediction simulation to the primary sequence information input by the input unit;
a prediction result comparison unit that compares a fragment structure prediction result of the fragment structure prediction program allowed to execute by the fragment structure prediction program execution unit with the tertiary structure data acquired by the tertiary structure data acquisition unit;
a frustration calculation unit that calculates a frustration of a local part of the primary sequence information on the target protein based on a comparison result of the prediction result comparison unit; and
an interaction site prediction unit that predicts an interaction site in the target protein based on the frustration of the local part calculated by the frustration calculation unit.

3. The interaction site prediction apparatus according to claim 1 or 2, further comprising:
a certainty factor information setting unit that sets certainty factor information indicating a certainty factor for the fragment structure prediction result of the fragment structure prediction program, wherein
the frustration calculation unit calculates the frustration of the local part based on the certainty factor information set by the certainty factor information setting unit and on the comparison result.

4. An interaction site prediction method comprising:
an input step that inputs primary sequence information on a target protein;
a fragment structure prediction program execution step that allows a fragment structure prediction program for predicting a fragment structure of the target protein from the primary sequence information on the target protein to execute a fragment structure prediction simulation to the primary sequence information input by the input step;
a prediction result comparison step that compares a fragment structure prediction result of the fragment structure prediction program allowed to execute by the fragment structure prediction program execution step with the fragment structure prediction result of the other fragment structure prediction program;
a frustration calculation step that calculates a frustration of a local part of the primary sequence information on the target protein based on a comparison result of the prediction result comparison step; and
an interaction site prediction step that predicts an interaction site in the target protein based on the frustration of the local part calculated by the frustration calculation step.

5. An interaction site prediction method comprising:
an input step that inputs primary sequence information on a target protein;
a tertiary structure data acquisition step that acquires tertiary structure data on the target protein;
a fragment structure prediction program execution step that allows a fragment structure prediction program for predicting a fragment structure of the target protein from the primary sequence information on the target protein to execute a fragment structure prediction simulation to the primary sequence information input by the input step;
a prediction result comparison step that compares a fragment structure prediction result of the fragment structure prediction program allowed to execute by the fragment structure prediction program execution step with the tertiary structure data acquired by the tertiary structure data acquisition step;
a frustration calculation step that calculates a frustration of a local part of the primary sequence information on the target protein based on a comparison result of the prediction result comparison step; and
an interaction site prediction step that predicts an interaction site in the target protein based on the frustration of the local part calculated by the frustration calculation step.

6. The interaction site prediction method according to claim 4 or 5, further comprising:
a certainty factor information setting step that sets certainty factor information indicating a certainty factor for the fragment structure prediction result of the fragment structure prediction program, wherein
the frustration calculation step calculates the frustration of the local part based on the certainty factor information set by the certainty factor information setting step and on the comparison result.

7. A computer program that makes a computer to execute an interaction site prediction method comprising:
an input step that inputs primary sequence information on a target protein;
a fragment structure prediction program execution step that allows a fragment structure prediction program for predicting a fragment structure of the target protein from the primary sequence information on the target protein to execute a fragment structure prediction simulation to the primary sequence information input by the input step;
a prediction result comparison step that compares a fragment structure prediction result of the fragment structure prediction program allowed to execute by the fragment structure prediction program execution step with the fragment structure prediction result of the other fragment structure prediction program;
a frustration calculation step that calculates a frustration of a local part of the primary sequence information on the target protein based on a comparison result of the prediction result comparison step; and
an interaction site prediction step that predicts an interaction site in the target protein based on the frustration of the local part calculated by the frustration calculation step.

8. A computer program that makes a computer to execute an interaction site prediction method comprising:
an input step that inputs primary sequence information on a target protein;
a tertiary structure data acquisition step that acquires tertiary structure data on the target protein;
a fragment structure prediction program execution step that allows a fragment structure prediction program for predicting a fragment structure of the target protein from the primary sequence information on the target protein to execute a fragment structure prediction simulation to the primary sequence information input by the input step;
a prediction result comparison step that compares a fragment structure prediction result of the fragment structure prediction program allowed to execute by the fragment structure prediction program execution step with the tertiary structure data acquired by the tertiary structure data acquisition step;
a frustration calculation step that calculates a frustration of a local part of the primary sequence information on the target protein based on a comparison result of the prediction result comparison step; and
an interaction site prediction step that predicts an interaction site in the target protein based on the frustration of the local part calculated by the frustration calculation step.

9. The program comprising according to claim 7 or 8, further comprising:
a certainty factor information setting step that sets certainty factor information indicating a certainty factor for the fragment structure prediction result of the fragment structure prediction program, wherein
the frustration calculation step calculates the frustration of the local part based on the certainty factor information set by the certainty factor information setting step and on the comparison result.

10. A computer readable recording medium storing a computer program that makes a computer to execute an interaction site prediction method comprising:
an input step that inputs primary sequence information on a target protein;
a fragment structure prediction program execution step that allows a fragment structure prediction program for predicting a fragment structure of the target protein from the primary sequence information on the target protein to execute a fragment structure prediction simulation to the primary sequence information input by the input step;
a prediction result comparison step that compares a fragment structure prediction result of the fragment structure prediction program allowed to execute by the fragment structure prediction program execution step with the fragment structure prediction result of the other fragment structure prediction program;
a frustration calculation step that calculates a frustration of a local part of the primary sequence information on the target protein based on a comparison result of the prediction result comparison step; and
an interaction site prediction step that predicts an interaction site in the target protein based on the frustration of the local part. calculated by the frustration calculation step.

11. A computer readable recording medium storing a computer program that makes a computer to execute an interaction site prediction method comprising:
an input step that inputs primary sequence information on a target protein;
a tertiary structure data acquisition step that acquires tertiary structure data on the target protein;
a fragment structure prediction program execution step that allows a fragment structure prediction program for predicting a fragment structure of the target protein from the primary sequence information on the target protein to execute a fragment structure prediction simulation to the primary sequence information input by the input step;
a prediction result comparison step that compares a fragment structure prediction result of the fragment structure prediction program allowed to execute by the fragment structure prediction program execution step with the tertiary structure data acquired by the tertiary structure data acquisition step;
a frustration calculation step that calculates a frustration of a local part of the primary sequence information on the target protein based on a comparison result of the prediction result comparison step; and
an interaction site prediction step that predicts an interaction site in the target protein based on the frustration of the local part calculated by the frustration calculation step.

12. The computer readable recording medium according to claim 10 or 11, further comprising:
a certainty factor information setting step that sets certainty factor information indicating a certainty factor for the fragment structure prediction result of the fragment structure prediction program, wherein
the frustration calculation step calculates the frustration of the local part based on the certainty factor information set by the certainty factor information setting step and on the comparison result.
